# NEUE EUROPÄISCHE PATENTSCHRIFT

(11) **EP 2 051 781 B2**
(45) Veröffentlichungstag und Bekanntmachung des Hinweises auf die Entscheidung über den Einspruch: **02.05.2018**
(45) Hinweis auf die Patenterteilung: 02.12.2015
(21) Anmeldenummer: 07786631.7
(22) Anmeldetag: 09.08.2007
(51) Int. Cl.: A61Q 19/00, A61Q 5/00, A61K 8/37, C07C 69/003, C07C 69/24, C07C 69/533, C07C 69/58, C07C 69/587, C07C 69/593, C07C 69/602, C07C 69/606

(54) **KOSMETISCHE ZUSAMMENSETZUNGEN ENTHALTEND ESTER AUF BASIS VON 2-BUTYL-OCTANOL**
COSMETIC COMPOSITIONS CONTAINING ESTER OBTAINED FROM 2-BUTYL-1-OCTANOL
COMPOSITIONS COSMÉTIQUES CONTENANT UN ESTER OBTENU À PARTIR DU 2-BUTYL-1-OCTANOL

(30) Priorität: 18.08.2006 EP 06017217
(43) Veröffentlichungstag der Anmeldung: 29.04.2009
(73) Patentinhaber: Cognis IP Management GmbH, 40589 Dusseldorf (DE)
(72) Erfinder: ANSMANN, Achim, 40699 Erkrath (DE); DIERKER, Markus, 40597 Düsseldorf (DE); WEICHOLD, Catherine, 52062 Aachen (DE)
(74) Vertreter: BASF IP Association
(86) Internationale Anmeldenummer: PCT/EP2007/007037
(87) Internationale Veröffentlichungsnummer: WO 2008/019794

(56) Entgegenhaltungen:
- EP-A- 0 930 063
- WO-A-01/01949
- WO-A-2005/009404
- DE-A1- 10 239 712
- DE-A1- 19 742 275
- GB-A- 1 121 543
- US-A- 2 014 310
- US-A- 5 474 714
- US-A1- 2005 240 040
- US-B1- 6 485 713
- US-B1- 6 827 220
- DATABASE WPI Week 200357 Derwent Publications Ltd., London, GB; AN 2003-601167 XP002417725 -& JP 2003 034795 A (SANYO CHEM IND LTD) 7. Februar 2003 (2003-02-07)
- G. KNOTHE: "Synthesis, Mass Spectrometry, and Nuclear Magnetic Resonance Characterization of Di-Guerbet Esters" J. AM. OIL CHEM. SOC., Bd. 75, Nr. 12, 1998, Seiten 1861-1866, XP001248929
- G. KNOTHE: "Characterization of Esters of Fatty Acids and Dicarboxylic Acids with Guerbet Alcohols" J. AM. OIL CHEM. SOC., Bd. 78, Nr. 5, 2001, Seiten 537-540, XP001248928 in der Anmeldung erwähnt

## Beschreibung

### Gebiet der Erfindung

Die Erfindung betrifft die Verwendung von Estern des 2-Butyl-1-octanols in kosmetischen und/oder pharmazeutischen Zubereitungen sowie kosmetische und/oder pharmazeutische Zusammensetzungen, die diese Ester enthalten.

### Stand der Technik

Im Bereich kosmetischer Emulsionen für die Haut- und Haarpflege werden vom Verbraucher eine Vielzahl von Anforderungen gestellt: Abgesehen von den reinigenden und pflegenden Effekten, die den Anwendungszweck bestimmen, wird Wert auf so unterschiedliche Parameter wie höchstmögliche dermatologische Verträglichkeit, gute rückfettende Eigenschaften, elegantes Erscheinungsbild, optimaler sensorischer Eindruck und Lagerstabilität gelegt.

Zubereitungen, die zur Reinigung und Pflege der menschlichen Haut und der Haare eingesetzt werden, enthalten in der Regel neben einer Reihe von oberflächenaktiven Substanzen, vor allem Ölkörper und Wasser. Als Ölkörper/Emollients werden beispielsweise Kohlenwasserstoffe, Esteröle sowie pflanzliche und tierische Öle/Fette/Wachse eingesetzt. Um die hohen Anforderungen des Marktes bezüglich sensorischer Eigenschaften und optimaler dermatologischer Verträglichkeit zu erfüllen, werden kontinuierlich neue Ölkörper und Emulgator-Gemische entwickelt und getestet. Die Verwendung von Esterölen in der Kosmetik ist seit langem bekannt. Wegen ihrer Bedeutung werden auch kontinuierlich neue Verfahren zu deren Herstellung entwickelt. Insbesondere verzweigte Esteröle vermitteln ein "leichteres" Hautgefühlt und werden daher intensiv untersucht. Der Einsatz von 2-Methyl-1,3-propanediolmonoestern ist beispielsweise Gegenstand der DE 101 60 681, der Einsatz von 2-Methyl-1,3-propanedioldiestern wird in der DE 101 60 682 beschrieben.

DE 19742275 beschreibt die Verwendung von verzweigtkettigen Estern als antibakterielle, antimikrotische, antiparasitäre oder antivirale Wirkstoffe. Es wird 2-Butyloctyl-2-butyloctanoat als verzweigtkettiger Ester genannt.

US 2,014,310 beschreibt Ester des 2-Butyloctanols. Insbesondere werden Ester beschrieben, die aus der Umsetzung von 2-Butyloctanol mit Carbonsäuren entstehen. Als Carbonsäuren werden Phtalsäureanhydrid, Salicylsäure und Bernsteinsäure genannt. Diese Ester werden als Weichmacher in Lacken verwendet.

EP 0930063 A2 beschreibt Hautpflegezusammensetzungen und Sonnenschutzmittel. Diese Zusammensetzungen enthalten cyclische Ester.

Aus US 6,485,713 ist ein Sonnenschutzmittel bekannt, das einen C4-Dicarbonsäureester enthält.

DE 10239712 A1 beschreibt eine kosmetische Zusammensetzung auf Basis ölhaltiger Mikroemulsionen. Die verwendeten Ester enthalten eine cyclische Carbonsäureeinheit.

WO 2005/009404 A1 beschreibt kosmetische Zusammensetzungen auf Basis von Gase enthaltenden Lipid/Wachsgemischen. Die verwendeten Ester sind mit cyclischen Carbonsäuren hergestellt.

Aus WO 01/01949 sind Reinigungszusammensetzungen bekannt, die im kosmetischen Bereich eingesetzt werden. Die in diesen Zusammensetzungen enthaltenden Ester enthalten eine cyclische Carbonsäureeinheit.
Aus WO 9515743 sind Fettalkoholester-Mischungen auf Basis von Guerbet-Alkoholen bekannt, die in kosmetischen und pharmazeutischen Zubereitungen eingesetzt werden.
Die DD8980 beschreibt ebenfalls Fettalkoholester-Mischungen, die in kosmetischen und pharmazeutischen Zubereitungen eingesetzt werden.

Aufgabe der vorliegenden Erfindung wares, neue, vorzugsweise bei 20°C flüssige Esteröle für kosmetische Applikationen zur Verfügung zu stellen, die bezüglich der sensorischen Eigenschaften (Leichtigkeit, "nicht-fettendes Hautgefühl", Weichheit, Spreitvermögen, Absorption, Verteilbarkeit, Öligkeit) über ein verbessertes Profil verfügen und in eine Vielzahl kosmetischer Formulierungen einarbeitbar sind. Hierbei waren auch die Hydrolysestabilität der Ester sowie die Formulierbarkeit der Ester bei niedrigem pH-Wert von Interesse. Weiterhin sollten die Ester sowohl in W/O als auch in O/W Formulierungen einarbeitbar sein. Weiterhin sollten die Ester insbesondere mit kristallinen UV-Filtern, Pigmenten, Antiperspirantien Salzen sowie Silikonen kompatibel sein. Überraschenderweise wurde gefunden, dass Ester des 2-Butyl-1-octanols zu sensorisch leichten Produkten führen. Einige dieser Ester sind beschrieben von Knothe G., JAOCS, Vol 78, No 5, 2001, p 537-540. Die dort beschriebenen Ester werden als mögliche Additive für Biodiesel beschrieben.

### Beschreibung der Erfindung

Gegenstand der Erfindung ist die Verwendung von Estern des 2-Butyl-1-octanols mit acyclischen C6-C10-Carbonsäuren in kosmetischen und/oder pharmazeutischen Zubereitungen.

Überaschenderweise sind Ester des 2-Butyl-1-octanols besonders gut für kosmetische Formulierungen geeignet, insbesondere für Formulierungen, bei denen es auf ein "leichtes" Hautgefühl ankommt. Die Ester lassen sich sehr gut in verschiedene Formulierungen einarbeiten. Je nach Kettenlänge, Verzweigung und Anzahl der Doppelbindungen erhält man flüssige Substanzgemische, die sich entsprechend als Ölkörper oder Konsistenzgeber eignen. Erfindungsgemäß kann ein einziger 2-Butyl-1-octyl-C6-C10-Carbonsäureestereingesetzt werden oder ein beliebiges Gemisch.

Gegenstand der Erfindung ist insbesondere die Verwendung von Estern des 2-Butyl-1-octanols mit acyclischen C6-C10-Carbonsäuren in kosmetischen und/oder pharmazeutischen Zubereitungen zur Benetzung oder Imprägnierung oder Beschichtung von Gebrauchs- und/oder Hygienetüchern, die zur Körperreinigung und/oder zur Körperpflege eingesetzt werden.

Die Begriffe "2-Butyl-1-octanol" und "2-Butyloctanol" werden in der vorliegenden Beschreibung synonym verwendet, ebenso wie die Präfixe "2-Butyl-1-octyl" und "2-Butyloctyl".

Die Verwendung von Estern des 2-Butyl-1-octanols mit gesättigten acyclischen Carbonsäuren ist erfindungsgemäß bevorzugt.

Die Verwendung von Estern des 2-Butyl-1-octanols mit acyclischen linearen, unverzweigten Carbonsäuren ist erfindungsgemäß bevorzugt.

Der Begriff "CX Carbonsäuren" umfasst Carbonsäuren mit einer Gesamtkohlenstoff Anzahl von X, also z.B. "C8 Carbonsäuren" umfasst alle Carbonsäuren, welche eine Gesamtkohlenstoffzahl von 8 haben, wie beispielsweise n-Octansäure, Isooctansäuren oder Methylheptansäuren.

Der Begriff "Carbonsäure" bezeichnet im Rahmen der vorliegenden Erfindung "Monocarbonsäuren".

Die sensorische Prüfung der erfindungsgemäßen Ester zeigt eine signifikante Verbesserung der Sensorik - insbesondere bezüglich der Spreitung gegenüber bekannten Emollients (z.B. verschiedene andere Esteröle oder Dialkylcarbonate).

Als Carbonsäuren können lineare oder verzweigte, gesättigte oder ungesättigte acyclische Carbonsäuren eingesetzt werden.

Erfindungsgemäß verwendete sind Ester des 2-Butyl-1-octanols mit beispielsweise (in Klammern Trivialnamen der Säuren) Hexansäure (Capronsäure), Heptansäure, Octansäure (Caprylsäure), i-Octansäure wie z.B. insbesondere 2-Ethylbutyl-2-ethylhexansäureester, aber auch 2-Ethylbutyl-3-ethylhexansäureester, 2-Ethylbutyl-4-ethylhexansäureester, 2-Ethylbutyl-5-ethylhexansäureester sowie technische Gemische von verzweigten Octansäuren, wie sie beispielsweise unter dem Handelsnamen Cekanoic® C8 von der Fa. Exxon vertrieben werden. Nonansäure (Pelargonsäure, Nonylsäure), Decansäure (Caprinsäure), i-Decansäuren, wie z.B. Trimethylheptansäure(Neodecansäure, Isodecansäure) sowie technische Gemische verzweigter Decansäuren, wie sich beispielsweise unter dem Handelsnamen Cekanoic® C 10 von der Fa. Exxon vertrieben werden.

Die Erfindung umfasst sowohl einzelne Ester aus auch Mischungen von verschiedenen Estern.
Eine Ausführungsform der Erfindung betrifft Ester des 2-Butyl-1-octanols mit verzweigten Carbonsäuren. Unter dem Begriff "i-Säure" mit XC-Atomen im Sinne der Erfindung sind alle verzweigten Carbonsäuren zu verstehen, welche in Summe X C-Atome enthalten. So z.B. Methyl-, Ethyl- oder Propyl- verzweigte, gegebenenfalls mehrfach verzweigte Carbonsäuren. In einer besonderen Ausführungsform wird die Untergruppe der gegebenenfalls mehrfachmethylverzweigten Carbonsäuren eingesetzt (= Iso-Säuren).

Bevorzugt sind die folgenden Ester: 2-Butyloctyl-n-nonansäureester und 2-Butyloctyl-i-nonansäureester,

Ein Gegenstand der Erfindung betrifft 2-Butyloctyl-n-nonansäureester. Ein Gegenstand der Erfindung betrifft 2-Butyloctyl-i-nonansäureester.

Überraschenderweise hat sich gezeigt, dass sich die Ester besonders eignen für die Verwendung in kosmetischen und/oder pharmazeutischen Zubereitungen.

Beschrieben wird auch ein Verfahren zur Herstellung der Ester, wobei man eine Mischung enthaltend 2-Butyloctanol und die entsprechende Säure umsetzt.

Beschrieben wird ein Verfahren zur Herstellung der Ester, wobei man eine Mischung enthaltend 2-Butyloctanol und mindestens eine acyclische C6 bis C10 Carbonsäure umsetzt.

Bevorzugt wird eine Mischung enthaltend 2-Butyloctanol und mindestens eine lineare, unverzweigte C6 bis C10 Dicarbonsäure umgesetzt.

Das Verfahren umfasst ebenfalls die Herstellung von Estergemischen, bei dem man 2-Butyloctanol zusammen mit den entsprechenden Säuregemischen umsetzt.

Das Verfahren umfasst ebenfalls die Herstellung von gemischten Estern des 2-Butyloctanol mit mindestens einer linearen, unverzweigten C6 bis C10 Dicarbonsäure, bei dem man ein Gemisch aus 2-Butyloctanol,
mindestens einer linearen, unverzweigten C6 bis C10 Dicarbonsäure und mindestens einen weiteren Alkohol der allgemeinen Formel R-OH, wobei R für einen gesättigten, linearen oder verzweigten, Alkylrest mit 1 bis 12 C-Atomen steht, umsetzt.

Man kann die Mischung enthaltend Alkohol und die entsprechende Säure unter Zusatz eines Veresterungskatalysators umsetzen.

Beispielsweise wird die Mischung enthaltend Alkohol und die entsprechende Säure erhitzt, das entstehende Wasser kontinuierlich abgeführt und das Rohprodukt danach destilliert. Das Verfahren kann unter Zusatz eines Veresterungskatalysators, z. B. säurekatalytisch oder basenkatalytisch durch geführt werden. In einer bevorzugten Ausführungsform wird das Verfahren ohne Zusatz von Lösungsmitteln durchgeführt, vorzugsweise mit Edukten, die möglichst wasserfrei sind. In einer bevorzugten Ausführungsform des Verfahrens wird ein Zinn-Katalysator einsetzt. Als Zinn-Katalysatoren geeignet sind beispielsweise Zinnoxalat (z. B. Fascat® 2001), Zinnoxid (SnO, Fascat® 2000) sowie Zinn-IV-Katalysatoren wie Dibutylzinndiacetat Fascat® 4200), Dibutylzinnoxid (Fascat® 4201), und Dibutylzinnlaurat (Fascat® 4202) oder Zinnoxid (SnO), die ehemals von Atofina und gegenwärtig von Arkema vermarktet werden.

Vorzugsweise wird die Veresterung bei Temperaturen zwischen 100-300°C, insbesondere 200-250°C durchführt.

Als Katalysator kann mindestens ein Enzym eingesetzt werden. Als Enzym eignen sich alle dem Fachmann bekannten Enzyme oder Enzymmischungen, welche in der Lage sind die Veresterung von Alkohol und Säure zu katalysieren, exemplarisch seien genannt Lipasen, Acyltransferasen und Esterasen. Die enzymatisch katalysierte Veresterung wird üblicherweise bei Temperaturen von 20 bis 100 °C, vorzugsweise 40 bis 80 °C durchgeführt.

Beschrieben wird ein Verfahren zur Herstellung der Ester, wobei man eine Mischung enthaltend 2-Butyloctanol und den Methylester der entsprechenden Säure unter Zusatz eines Umesterungskatalysators umsetzt.

Das Verfahren umfasst ebenfalls die Herstellung von Estergemischen, bei dem man 2-Butyloctanol zusammen mit den entsprechenden Mischungen der Methylester der Säuren unter Zusatz eines Umesterungskatalysators umsetzt.

Beispielsweise wird die Mischung enthaltend Alkohol und den Methylester der entsprechenden Säure unter Zusatz des Veresterungskatalysators erhitzt, das entstehende Wasser kontinuierlich abgeführt und das Rohprodukt danach destilliert. In einer bevorzugten Ausführungsform wird das Verfahren ohne Zusatz von Lösungsmitteln durchgeführt, vorzugsweise mit Edukten, die möglichst wasserfrei sind.

Vorzugsweise wird die Veresterung bei Temperaturen zwischen 100- 300°C, insbesondere 200-250°C durchführt. Als Umesterungskatalysator können alle dem Fachmann bekannten Umesterungskatalysatoren eingesetzt, werden, vorzugsweise wird als Umesterungskatalysator Natriummethylat oder Tetraalkyltitanat eingesetzt.

Beispielsweise wird als Katalysator mindestens ein Enzym eingesetzt. Als Enzym eignen sich alle dem Fachmann bekannten Enzyme oder Enzymmischungen, welche in der Lage sind die Umesterung von Alkohol und Säuremethylester zu katalysieren, exemplarisch seien genannt Lipasen, Acyltransferasen und Esterasen. Die enzymatisch katalysierte Veresterung wird üblicherweise bei Temperaturen von 20 bis 100 °C, vorzugsweise 40 bis 80 °C durchgeführt.

### Kosmetische/pharmazeutische Zubereitungen

Die 2-Butyl-1-octylester erlauben die Herstellung stabiler kosmetischer und pharmazeutischer Emulsionen mit besonders leichtem Hautgefühl.

Ein weiterer Gegenstand der vorliegenden Erfindung sind daher kosmetische und/oder pharmazeutische Zubereitungen enthaltend
(a) wenigstens einen Ester des 2-Butyl-1-octanols mit linearen C6-C10-Carbonsäuren
(b) wenigstens einen Emulgator (b-1) und/oder Tensid (b-2) und/oder Wachskomponente (b-3) und/oder Polymer (b-4) und/oder einen weiteren Ölkörper (b-5).

Ein Gegenstand der vorliegenden Erfindung sind kosmetische und/oder pharmazeutische Zubereitungen enthaltend
a) wenigstens einen Ester des 2-Butyl-1-octanols mit linearen C6-C10-Carbonsäuren
b-1) wenigstens einen Emulgator

Ein Gegenstand der vorliegenden Erfindung sind kosmetische und/oder pharmazeutische Zubereitungen enthaltend
a) wenigstens einen Ester des 2-Butyl-1-octanols mit linearen C6-C10-Carbonsäuren
b-2) wenigstens ein Tensid.

Ein Gegenstand der vorliegenden Erfindung sind kosmetische und/oder pharmazeutische Zubereitungen enthaltend
a) wenigstens einen Ester des 2-Butyl-1-octanols mit linearen C6-C10-Carbonsäuren
b-3) wenigstens eine Wachskomponente

Ein Gegenstand der vorliegenden Erfindung sind kosmetische und/oder pharmazeutische Zubereitungen enthaltend
a) wenigstens einen Ester des 2-Butyl-1-octanols mit linearen C6-C10-Carbonsäuren
b) b-4) wenigstens ein Polymer.

Ein Gegenstand der vorliegenden Erfindung sind kosmetische und/oder pharmazeutische Zubereitungen enthaltend
a) wenigstens einen Ester des 2-Butyl-1-octanols mit linearen C6-C10-Carbonsäuren
b-5) wenigstens einen weiteren Ölkörper.

Vorzugsweise enthalten die erfindungsgemäßen Zubereitungen 0,1 bis 80, insbesondere 0,5 bis 70, vorzugsweise 0,75 bis 60 Gew.-%, insbesondere 1 bis 50 Gew.-%, bevorzugt 1 - 40 Gew.-% wenigstens eines Esters des 2-Butyloctanols mit linearen C6-C10-Carbonsäuren.

Ein weiterer Gegenstand der Erfindung sind kosmetische und/oder pharmazeutische Zubereitungen enthaltend
a) 0,1 bis 80 Gew.-%, insbesondere 0,1 bis 70, bevorzugt 0,1 bis 60, insbesondere 0,1 bis 50 Gew.-%, bevorzugt 0,1 - 40 Gew.-% wenigstens eines Esters des 2-Butyl-1-octanols mit linearen C6-C10-Carbonsäuren
b) 0,1 - 20 Gew.-% Emulgator (b-1) und/oderTensid(b-2) und/oder Wachskomponente(b-3) und/oder Polymer (b-4)
   b-5) 0,1 - 40 Gew.-% weiterer Ölkörper und
c) 0 - 98 Gew.-% Wasser.

Die erfindungsgemäßen Zubereitungen enthalten mindestens 0,1, insbesondere mindestens 0,5, insbesondere mindestens 0,75, bevorzugt mindestens 1, bevorzugt mindestens 5 Gew.-% eines oder mehrerer Ester (a).

Alle Gew.-% Angaben beziehen sich auf Gew.-% bezogen auf die kosmetische und/oder pharmazeutische Zubereitung.

Die erfindungsgemäßen Zubereitungen enthalten bevorzugt Ester des 2-Butyl-1-octanols mit aliphatischen Carbonsäuren.

Im Sinne der Erfindung sind Zubereitungen bevorzugt, die Ester des 2-Butyl-1-octanols mit linearen unverzweigten Carbonsäuren enthalten.

Die erfindungsgemäßen Zubereitungen enthalten bevorzugt Ester des 2-Butyl-1-octanols mit gesättigten Carbonsäuren.

Die erfindungsgemäßen Zubereitungen können sowohl einzelne Ester aus auch Mischungen verschiedener Ester enthalten.

In einer bevorzugten Ausführungsform der Erfindung enthalten die Zubereitungen mindestens einen Ester ausgewählt aus der Gruppe bestehend aus 2-Butyloctyl-n-nonansäureester.

Eine weitere bevorzugte Ausführungsform der kosmetischen und/oder pharmazeutischen Zubereitungen enthält (a) 0,1 - 80, insbesondere 0,1 bis 70, vorzugsweise 0,1 bis 60, bevorzugt 0,1 bis 50 Gew.-% wenigstens eines Esters des 2-Butyl-1-octanols mit linearen C6-C10-Carbonsäuren (b) 0,1 - 20 Gew.-% Emulgatoren (b-1) und/oder Tenside (b-2) und/oder Wachskomponenten (b-3) und/oder Polymere (b-4), und 0,1 - 40 Gew.-% weiterer Ölkörper (b-5) und (d) 0 - 98 Gew.-% Wasser.

Die erfindungsgemäßen Zusammensetzungen eignen sich als Basis in allen kosmetischen Mitteln zur Körperpflege und -reinigung wie z.B. Körperöl, Babyöl, Körpermilch, Cremes, Lotionen, sprühbare Emulsionen, Sonnenschutzmittel. Antitranspirantien, Flüssig- und Stückseifen etc. eingearbeitet werden. Sie lassen sich auch in tensidhaltigen Formulierungen wie z.B. Schaum- und Duschbädem, Haarshampoos und Pflegespülungen einsetzen. Sie lassen sich als Pflegekomponente auf Tissues Papiere, Wipes, Vlies-Produkte, Schwämme, Puffs, Pflaster und Bandagen applizieren, die ihren Einsatz im Bereich der Hygiene und Pflege finden (Feuchttücher zur Baby-Hygiene und Baby-Pflege, Reinigungstücher, Gesichtreinigungstücher, Hautpflegetücher, Pflegetücher mit Wirkstoffen gegen die Hautalterung, Wipes mit Sonnenschutzformulierungen und Insektenrepellentien sowie Wipes zur dekorativen Kosmetik oder zum After-Sun-Treatment, Toiletten-Feuchttücher, Antitranspirant-Wipes, Windel, Taschentücher, Wet Wipes, Hygieneprodukte, Selbstbräunungs Wipes). Sie lassen sich u.a. auch in Zubereitungen zur Haarpflege, Haarreinigung oder Haarfärbung einsetzen.

Je nach Applikationszweck enthalten die kosmetischen Formulierungen eine Reihe weiterer Hilfs- und Zusatzstoffe, wie beispielsweise Tenside, weitere Ölkörper, Emulgatoren, Perlglanzwachse, Konsistenzgeber, Verdickungsmittel, Überfettungsmittel, Stabilisatoren, Polymere, Fette, Wachse, Lecithine, Phospholipide, biogene Wirkstoffe, UV-Lichtschutzfaktoren, Antioxidantien, Deodorantien, Antitranspirantien, Antischuppenmittel, Filmbildner, Quellmittel, Insektenrepeltentien, Selbstbräuner, Tyrosinaseinhibitoren (Depigmentierungsmittel), Hydrotrope, Solubilisatoren, Konservierungsmittel, Parfümöle, Farbstoffe etc., die nachstehend exemplarisch aufgelistet sind.

### Emulgator b-1)

In einer Ausführungsform der Erfindung enthalten die erfindungsgemäßen Zubereitungen mindestens einen Emulgator. Die erfindungsgemäßen Zusammensetzungen enthalten den/die Emulgator(en) in einer Menge von 0 bis 40 Gew.-%, vorzugsweise 0,1 bis 20 Gew.-% vorzugsweise 0,1 bis 15 Gew.-% und insbesondere 0,1 bis 10 Gew.-% bezogen auf das Gesamtgewicht der Zusammensetzung.

In einer Ausführungsform der Erfindung enthält die erfindungsgemäße Zubereitung mehr als einen Emulgator. Der Fachmann setzt in Abhängigkeit der übrigen Komponenten übliche Emulgator Systeme (wie z.B. Emulgator und Co-Emulgator) ein.

### Nicht-ionische Emulgatoren

Zur Gruppe der nicht-ionischen Emulgatoren gehören beispielsweise:
(1) Anlagerungsprodukte von 2 bis 50 Mol Ethylenoxid und/oder 1 bis 20 Mol Propylenoxid an lineare Fettalkohole mit 8 bis 40 C-Atomen, an Fettsäuren mit 12 bis 40 C-Atomen und an Alkylphenole mit 8 bis 15 C-Atomen in der Alkylgruppe.
(2) C₁₂- bis C₁₈-Fettsäuremono- und -diester von Anlagerungsprodukten von 1 bis 50 Mol Ethylenoxid an Glycerin.
(3) Sorbitanmono- und -diester von gesättigten und ungesättigten Fettsäuren mit 6 bis 22 Kohlenstoffatomen und deren Ethylenoxidanlagerungsprodukte.
(4) Alkylmono- und -oligoglycoside mit 8 bis 22 Kohlenstoffatomen im Alkylrest und deren ethoxylierte Analoga.
(5) Anlagerungsprodukte von 7 bis 60 Mol Ethylenoxid an Ricinusöl und/oder gehärtetes Ricinusöl.
(6) Polyol- und insbesondere Polyglycerinester, wie z. B. Polyolpoly-12-hydroxystearate, Polyglycerinpolyricinoleat, Polyglycerindiisostearat oder Polyglycerindimerat. Ebenfalls geeignet sind Gemische von Verbindungen aus mehreren dieser Substanzklassen.
(7) Anlagerungsprodukte von 2 bis 15 Mol Ethylenoxid an Ricinusöl und/oder gehärtetes Ricinusöl.
(8) Partialester auf Basis linearer, verzweigter, ungesättigter bzw. gesättigter C₆- bis C₂₂-Fettsäuren, Ricinolsäure sowie 12-Hydroxystearinsäure und Polyglycerin, Pentaerythrit, Dipentaerythrit, Zuckeralkohole (z. B. Sorbit), Alkylglucoside (z. B. Methylglucosid, Butylglucosid, Laurylglucosid) sowie Polyglucoside (z. B. Cellulose), oder Mischester wie z. B. Glycerylstearatcitrat und Glycerylstearatlactat.
(9) Polysiloxan-Polyalkyl-Polyether-Copolymere bzw. entsprechende Derivate.
(10) Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.

Die Anlagerungsprodukte von Ethylenoxid und/oder von Propylenoxid an Fettalkohole, Fettsäuren, Alkylphenole, Glycerin mono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht.

Je nach Ethoxylierungsgrad handelt es sich um W/O- oder O/W-Emulgatoren. C_{12/18}-Fettsäuremono-und -diester von Anlagerungsprodukten von Ethylenoxid an Glycerin sind als Rückfettungsmittel für kosmetische Zubereitungen bekannt.

Erfindungsgemäß besonders gut geeignete und milde Emulgatoren sind Polyolpoly-12-hydroxystearate und Abmischungen davon, welche beispielsweise unter den Marken "Dehymuls® PGPH" (W/O-Emulgator) oder "Eumulgin® VL 75" (Abmischung mit Coco Glucosides im Gewichtsverhältnis 1:1, O/W-Emulgator) oder Dehymuls® SBL (W/O-Emulgator) von der Cognis Deutschland GmbH vertrieben werden. In diesem Zusammenhang sei insbesondere auf das Europäische Patent EP 0 766 661 B1 verwiesen. Die Polyolkomponente dieser Emulgatoren kann sich von Stoffen ableiten, die über mindestens zwei, vorzugsweise 3 bis 12 und insbesondere 3 bis 8 Hydroxylgruppen und 2 bis 12 Kohlenstoffatome verfügen.

Als lipophile W/O-Emulgatoren eignen sich prinzipiell Emulgatoren mit einem HLB-Wert von 1 bits 8, die in zahlreichen Tabellenwerken zusammengefasst und dem Fachmann bekannt sind. Einige dieser Emulgatoren sind beispielsweise in Kirk-Othmer, "Encyclopedia of Chemical Technology", 3. Aufl., 1979, Band 8, Seite 913, aufgelistet. Für ethoxylierte Produkte lässt sich der HLB-Wert auch nach folgender Formel berechnen: HLB = (100 - L) : 5, wobei L der Gewichtsanteil der lipophilen Gruppen, d. h. der Fettalkyl- oder Fettacylgruppen in Gewichtsprozent, in den Ethylenoxidaddukten ist.

Besonders vorteilhaft aus der Gruppe der W/O-Emulgatoren sind Partialester von Polyolen, insbesondere von C₄-C₆-Polyolen, wie beispielsweise Partialester des Pentaerythtits oder Zuckerestem, z. B. Saccharosedistearat, Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinaleat, Sorbitanmonohydroxystearat, Sorbitansesquihydroxystearat, Sorbitandihydroxystearat, Sorbitantrihydroxystearat, Sorbilanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Als Emulgatoren geeignet sind auch Anlagerungsprodukte von 1 bis 30, vorzugsweise 5 bis 10 Mol Ethylenoxid an die genannten Sorbitanester.

Je nach Formulierung kann es vorteilhaft sein, zusätzlich wenigstens einen Emulgator aus der Gruppe nicht-ionischer O/W-Emulgatoren (HLB-Wert: 8 - 18) und/oder Solubilisatoren einzusetzen. Hierbei handelt es sich beispielsweise um die bereits einleitend erwähnten Ethylenoxid-Addukte mit einem entsprechend hohen Ethoxylierungsgrad, z. B. 10 - 20 Ethylenoxid-Einheiten für O/W-Emulgatoren und 20 - 40 Ethylenoxid-Einheiten für sogenannte Solubilisatoren. Erfindungsgemäß besonders vorteilhaft als O/W-Emulgatoren sind Ceteareth-12 und PEG-20 Stearat. Als Solubilisatoren bevorzugt geeignet sind Eumulgin® HRE 40 (INCI: PEG-40 Hydrogenated Castor Oil), Eumulgin® HRE 60 (INCI: PEG-60 Hydrogenated Castor Oil), Eumulgin® L (INCI: PPG-1-PEG-9 Laurylglycolether), sowie Eumulgin® SML 20 (INCI: Polysorbat-20).

Nicht-ionische Emulgatoren aus der Gruppe der Alkyloligoglycoside sind besonders hautfreundlich und daher bevorzugt als O/W-Emulgatoren geeignet. C₈-C₂₂ Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 22 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomensationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt. Produkte, die unter der Bezeichnung Plantacare® zur Verfügung stehen, enthalten eine glucosidisch gebundene C₈-C₁₆-Alkylgruppe an einem Oligoglucosidrest, dessen mittlerer Oigomerisationsgrad bei 1 bis 2 liegt. Auch die vom Glucamin abgeleiteten Acylglucamide sind als nicht-ionische Emulgatoren geeignet. Erfindungsgemäß bevorzugt ist ein Produkt, das unter der Bezeichnung Emulgade® PL 68/50 von der Cognis Deutschland GmbH vertrieben und ein 1:1-Gemisch aus Alkylpolyglucosiden und Fettalkoholen darstellt. Erfindungsgemäß vorteilhaft einsetzbar ist auch ein Gemisch aus Lauryl Glucoside, Polyglyceryl-2-Dipolyhydroxystearate, Glycerin und Wasser, das unter der Bezeichnung Eumulgin® VL 75 im Handel ist.

Als Emulgatoren kommen weiterhin Substanzen, wie Lecithine und Phospholipide in Frage. Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### Tenside b-2)

In einer Ausführungsform der Erfindung enthalten die erfindungsgemäßen Zubereitungen mindestens ein Tensid. Als oberflächenaktive Stoffe können anionische, nichtionische, kationische und/oder amphotere bzw. zwitterionische Tenside enthalten sein. In tensidhaltigen kosmetischen Zubereitungen, wie beispielsweise Duschgelen, Schaumbädern, Shampoos etc. ist vorzugsweise wenigstens ein anionisches Tensid enthalten.

Die erfindungsgemäßen Zusammensetzungen enthalten den/die Tenside in einer Menge von 0 bis 40 Gew.-%., vorzugsweise 0 bis 20 Gew.-%. vorzugsweise 0,1 bis 15 Gew.-% und insbesondere 0,1 bis 10 Gew.-% bezogen auf das Gesamtgewicht der Zusammensetzung.

Typische Beispiele für nichtionische Tenside sind Fettalkoholpolyglycolether, Alkylphenolpolyglycolether, Fettsäurepolyglycolester, Fettsäureamidpolyglycolether, Fettamin-polyglycolether, alkoxylierte Triglyceride, Mischether bzw. Mischformale, gegebenenfalls partiell oxidierte Alk(en)yloligoglykoside bzw. Glucoronsäurederivate, Feftsäure-N-alkylglucamide, Proteinhydrolysate (insbesondere pftanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen.

Als **zwitterionische Tenside** werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acyl-aminopropyl-N,N-dimethylammoniumglycinate, beispielsweise das Kokosacylaminopropyldimethyl-ammoniumglycinat, und 2-Alkyl-3-carboxylmethyl-3-hydroxyethylimidazolin mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls, insbesondere als Co-Tenside geeignet, sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyl-iminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

**Anionische Tenside** sind gekennzeichnet durch eine wasserlöslich machende, anionische Gruppe wie z. B. eine Carboxylat-, Sulfat-, Sulfonat- oder Phosphat-Gruppe und einen lipophilen Rest. Hautverträgliche anionische Tenside sind dem Fachmann in großer Zahl aus einschlägigen Handbüchern bekannt und im Handel erhältlich. Es handelt sich dabei insbesondere um Alkylsulfate in Form ihrer Alkali-, Ammonium- oder Alkanolammoniumsalze, Alkylethersulfate, Alkylethercarboxylate, Acylisethionate, Acylsarkosinate, Acyltaurine mit linearen Alkyl- oder Acylgruppen mit 12 bis 18 C-Atomen sowie Sulfosuccinate und Acylglutamate in Form ihrer Alkali- oder Ammoniumsalze.

Als kationische Tenside sind insbesondere quartäre Ammoniumverbindungen verwendbar. Bevorzugt sind Ammoniumhalogenide, insbesondere Chloride und Bromide, wie Alkyltrimethylammoniumchloride, Dialkyldimethylammoniumchloride und Trialkylmethylammoniumchloride, z.B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weiterhin können die sehr gut biologisch abbaubaren quaternären Esterverbindungen, wie beispielsweise die unter dem Warenzeichen Stepantex® vertriebenen Dialkylammoniummethosulfate und Methylhydroxyalkyldialkoyloxyalkylammoniummethosulfate und die entsprechenden Produkte der Dehyquart®-Reihe, als kationische Tenside eingesetzt werden. Unter der Bezeichnung "Esterquats" werden im allgemeinen quatemierte Fettsäuretriethanolaminestersalze verstanden. Sie können den erfindungsgemäßen Zusammensetzungen einen besonderen Weichgriff verleihen. Es handelt sich dabei um bekannte Stoffe, die man nach den einschlägigen Methoden der organischen Chemie herstellt. Weitere erfindungsgemäß verwendbare kationische Tenside stellen die quaternisierten Proteinhydrolysate dar.

### Wachskomponente b-3)

In einer Ausführungsform der Erfindung enthalten die erfindungsgemäßen Zubereitungen mindestens eine Wachskomponente. Die erfindungsgemäßen Zusammensetzungen enthalten den/die Wachskomponente(n) in einer Menge von 0 bis 40 Gew.-%, insbesondere von 0 bis 20 Gew.-%, vorzugsweise 0,1 bis 15 Gew.-% und insbesondere 0,1 bis 10 Gew.-% bezogen auf das Gesamtgewicht der Zusammensetzung.

Unter dem Begriff Wachs werden üblicherweise alle natürlichen oder künstlich gewonnenen Stoffe und Stoffgemische mit folgenden Eigenschaften verstanden: sie sind von fester bis brüchig harter Konsistenz, grob bis feinkristallin, durchscheinend bis trüb und schmelzen oberhalb von 30°C ohne Zersetzung. Sie sind schon wenig oberhalb des Schmelzpunktes niedrigviskos und nicht fadenziehend und zeigen eine stark temperaturabhängige Konsistenz und Löslichkeit. Erfindungsgemäß einsetzbar ist eine Wachskomponente oder ein Gemisch von Wachskomponenten, die bei 30 C oder darüber schmelzen.

Als Wachse können erfindungsgemäß auch Fette und fettähnliche Substanzen mit wachsartiger Konsistenz eingesetzt werden, solange sie den geforderten Schmelzpunkt haben. Hierzu gehören u.a. Fette (Triglyceride), Mono- und Diglyceride, natürliche und synthetische Wachse, Fett- und Wachsalkohole, Fettsäuren; Ester von Fettalkoholen und. Fettsäuren sowie Fettsäureamide oder beliebige Gemische dieser Substanzen.

Unter Fetten versteht man Triacylglycerine, also die Dreifachester von Fettsäuren mit Glycerin. Bevorzugt enthalten sie gesättigte, unverzweigte und unsubstituierte Fettsäurereste. Hierbei kann es sich auch um Mischester, also um Dreifachester aus Glycerin mit verschiedenen Fettsäuren handeln. Erfindungsgemäß einsetzbar und als Konsistenzgeber besonders gut geeignet sind sogenannte gehärtete Fette und Öle, die durch Partialhydrierung gewonnen werden. Pflanzliche gehärtete Fette und Öle sind bevorzugt, z. B. gehärtetes Rizinusöl, Erdnußöl, Sojaöl, Rapsöl, Rübsamenöl, Baumwollsaatöl, Sojaöl, Sonnenblumenöl, Palmöl, Palmkemöl, Leinöl, Mandelöl, Maisöl, Olivenöl, Sesamöl, Kakaobutter und Kokosfett.

Geeignet sind u.a. die Dreifachester von Glycerin mit C12-C60-Fettsäuren und insbesondere C12-C36-Fettsäuren. Hierzu zählt gehärtetes Rizinusöl, ein Dreifachester aus Glycerin und einer Hydroxystearinsäure, der beispielsweise unter der Bezeichnung Cutina HR im Handel ist. Ebenso geeignet sind Glycerintristearat, Glycerintribehenat (z.B. Syncrowax HRC), Glycerintripalmitat oder die unter der Bezeichnung Syncrowax HGLC bekannten Triglycerid-Gemische, mit der Vorgabe, dass der Schmelzpunkt der Wachskomponente bzw. des Gemisches bei 30 °C oder darüber liegt.

Als Wachskomponenten sind erfindungsgemäß insbesondere Mono- und Diglyceride bzw. Mischungen dieser Partialglyceride einsetzbar. Zu den erfindungsgemäß einsetzbaren Glyceridgemischen zählen die von der Cognis Deutschland GmbH & Co. KG vermarkteten Produkte Novata AB und Novata B (Gemisch aus C12-C18-Mono-, Di- und Triglyceriden) sowie Cutina MD oder Cutina GMS (Glycerylstearat).

Zu den erfindungsgemäß als Wachskomponente einsetzbaren Fettalkoholen zählen die C12-C50-Fettalkohole. Die Fettalkohole können aus natürlichen Fetten, Ölen und Wachsen gewonnen werden, wie beispielsweise Myristylalkohol, 1-Pentadecanol, Cetylalkohol, 1-Heptadecanol, Stearylalkohol, 1-Nonadecanol, Arachidylalkohol, 1-Heneicasanol, Behenylalkohol, Brassidylalkohol, Lignocerylalkohol, Cerylalkohol oder Myricylalkohol. Erfindungsgemäß bevorzugt sind gesättigte unverzweigte Fettalkohole. Aber auch ungesättigte, verzweigte oder unverzweigte Fettalkohole können erfindungsgemäß als Wachskomponente verwendet werden, solange sie den geforderten Schmelzpunkt aufweisen. Erfindungsgemäß einsetzbar sind auch Fettalkoholschnitte, wie sie bei der Reduktion natürlich vorkommender Fette und Öle wie z. B. Rindertalg, Erdnußöl, Rüböl, Baumwollsaatöl, Sojaöl, Sonnenblumenöl, Palmkemöl, Leinöl, Rizinusöl, Maisöl, Rapsöl, Sesamöl, Kakaobutter und Kokosfett anfallen. Es können aber auch synthetische Alkohole, z. B. die linearen, geradzahligen Fettalkohole der Ziegler-Synthese (Alfole) oder die teilweise verzweigten Alkohole aus der Oxosynthese (Dobanole) verwendet werden. Erfindungsgemäß besonders bevorzugt geeignet sind C14-C22-Feftalkohole, die beispielsweise von der Cognis Deutschland GmbH unter der Bezeichnung Lanette 16 (C16-Alkohol). Lanette 14 (C14-Alkohol), Lafette O (C16/C18-Alkohol) und Lanette 22 (C18/C22-Alkohol) vermarktet werden. Fettalkohole verleihen den Zusammensetzungen ein trockeneres Hautgefühl als Triglyceride und sind daher gegenüber letzteren bevorzugt.

Als Wachskomponenten können auch C14-C40-Fettsäuren oder deren Gemische eingesetzt werden. Hierzu gehören beispielsweise Myristin-, Pentadecan-, Palmitin-, Margarin-, Stearin-, Nonadecan-, Arachin-, Behen-, Lignocerin-, Cerotin-, Melissin-, Eruca- und Elaeostearinsäure sowie substituierte Fettsäuren, wie z. B. 12-Hydroxystearinsäure, und die Amide oder Monoethanolamide der Fettsäuren, wobei diese Aufzählung beispielhaften und keinen beschränkenden Charakter hat.

Erfindungsgemäß verwendbar sind beispielsweise natürliche pflanzliche Wachse, wie Candelillawachs, Carnaubawachs, Japanwachs, Espartograswachs, Korkwachs, Guarumawachs, Reiskeimölwachs, Zuckerrohrwachs, Ouricurywachs, Montanwachs, Sonnenblumenwachs, Fruchtwachse wie Orangenwachse, Zitronenwachse, Grapefruitwachs, Lorbeerwachs (=Bayberrywax) und tierische Wachse, wie z. B. Bienenwachs, Schellackwachs, Walrat, Wollwachs und Bürzelfett. Im Sinne der Erfindung kann es vorteilhaft sein, hydrierte oder gehärtete Wachse einzusetzen. Zu den erfindungsgemäß verwendbaren natürlichen Wachsen zählen auch die Mineralwachse, wie z. B. Ceresin und Ozokerit oder die petrochemischen Wachse, wie z. B. Petrolatum, Paraffinwachse und Mikrowachse. Als Wachskomponente sind auch chemisch modifizierte Wachse, insbesondere die Hartwachse, wie z. B. Montanesterwachse, Sasolwachse und hydrierte Jojobawachse einsetzbar. Zu den synthetischen Wachsen, die erfindungsgemäß einsetzbar sind, zählen beispielsweise wachsartige Polyalkylenwachse und Polyethylenglycolwachse. Pflanzliche Wachse sind erfindungsgemäß bevorzugt.

Die Wachskomponente kann ebenso gewählt werden aus der Gruppe der Wachsester aus gesättigten und/oder ungesättigten, verzweigten und/oder unverzweigten Alkancarbonsäuren und gesättigten und/oder ungesättigten, verzweigten und/oder unverzweigten Alkoholen, aus der Gruppe der Ester aus aromatischen Carbonsäuren, Dicarbonsäuren, Tricarbonsäuren bzw. Hydroxycarbonsäuren (z. B. 12-Hydroxystearinsäure) und gesättigten und/oder ungesättigten, verzweigten und/oder unverzweigten Alkoholen, sowie ferner aus der Gruppe der Lactide langkettiger Hydroxycarbonsäuren. Beispiel solcher Ester sind die C16-C40-Alkylstearate, C20-C40-Alkylstearate (z. B. Kesterwachs K82H), C20-C40-Dialkylester von Dimersäuren, C18-C38-Alkylhydroxystearoylstearate oder C20-C40-Alkylerucate. Ferner sind C30-C50-Alkylbienenwachs, Tristearylcitrat, Trisostearylcitrat, Stearylheptanoat, Stearyloctanoat, Trilaurylcitrat, Ethylenglycoldipalmitat, Ethylenglycoldistearat, Ethylenglykoldl(12-hydroxystearat), Stearylstearat, Palmitylstearat, Stearylbehenat, Cetylester, Cetearylbehenat und Behenylbehenat einsetzbar.

### Polymere b-4)

In einer Ausführungsform der Erfindung enthalten die erfindungsgemäßen Zubereitungen mindestens ein Polymer. Die erfindungsgemäßen Zusammensetzungen enthalten das/die Polymere(n) in einer Menge von 0 bis 20 Gew.-%, vorzugsweise 0,1 bis 15 Gew.-% und insbesondere 0,1 bis 10 Gew.% bezogen auf das Gesamtgewicht der Zusammensetzung.

Geeignete **kationische Polymere** sind beispielsweise kationische Cellulosederivate, wie z. B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeplide, wie beispielsweise Lauryldimonium hydroxypropyl hydrolyzed collagen (Lamequat®L/Grünau), quaternierte Weizenpolypeptide, Polyethylenimin, kationische Siliconpolymere, wie z. B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z. B. Mirapol® A-15, Mirapol® AD-1, Mirapol® AZ-1 der Firma Miranol.

Als **anionische, zwitterionische, amphotere und nichtionische Polymere** kommen beispielsweise Vinylacetat/Crotonsäure-Copolymere, Vinylpyrrolidon/Vinylacrylat-Copolymere, Vinylacetat/Butylmaleat/Isobomylacrylat-Copolymere, Methylvinylether/Maleinsäureanhydrid-Copolymere und deren Ester, unvemetzte und mit Polyolen vernetzte Polyacrylsäuren, Acrylamidopropyltrimethylammoniumchlorid/ Acrylat-Copolymere, Octylacrylamid/Methylmethacrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/ Dimethylaminoethylmethacryla/Ninylcaprolactam-Terpolymere sowie gegebenenfalls derivatisierte Celluloseether und Silicone in Frage.

Als Polymere eigen sich ebenso Polysaccharide, insbesondere Xanthan-Gum, Guar-Guar, Agar-Agar, Alginate und Tylosen.

### Weitere Ölkörper b-5)

Körperpflegemittel, wie Cremes, Körperöle, Lotionen und Milchen, enthalten üblicherweise eine Reihe weiterer Ölkörper und Emollients, die dazu beitragen, die sensorischen Eigenschaften weiter zu optimieren. Die Ölkörper (Ester plus weitere Ölkörper) sind üblicherweise in einer Gesamtmenge von 0,1 - 80, insbesondere 0,5 bis 70, bevorzugt 1 bis 60, insbesondere 1 bis 50 Gew.-%, insbesondere 1 bis 40 Gew.-%, vorzugsweise 5 - 25 Gew.-% und insbesondere 5 - 15 Gew.-% enthalten. Die weiteren Ölkörper sind üblicherweise in einer Menge von 0,1 bis 40 Gew.-% enthalten Als weitere Ölkörper kommen beispielsweise Guerbetalkohole auf Basis von Fettalkoholen mit 6 bis 18, vorzugsweise 8 bis 10 Kohlenstoffatomen, in Frage sowie weitere, zusätzliche Ester wie Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenytstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von C₁₈-C₃₈-Alkylhydroxycarbonsäure mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol), Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₆- C₂₂-Dicarbonsäuren mit Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol® CC), Guerbelcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen(z.B. Finsolv® TN), lineare oder verzweigte, symmetrische oder unsymmetrische Dialkylether mit 6 bis 22 Kohlenstoffatomen pro Alkylgruppe, wie z. B. Dicaprylylether (Cetiol® OE), Ringöffnungsprodukte von epoxidierten Fettsäureestern mit Polyolen und Kohlenwasserstoffen oder deren Gemischen (Cetiol® DD).

### Weitere Inhaltsstoffe

Als **Verdickungsmittel** eignen sich beispielsweise Aerosil-Typen (hydrophile Kieselsäuren), Carboxymethylcellulose und Hydroxyethyl- und Hydroxypropylcellulose, Polyvinylalkohol, Polyvinylpyrrolidon und Bentonite wie z. B. Bentone® Gel VS-5PC (Rheox).

Unter **UV-Lichtschutzfaktoren** sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z. B. Wärme wieder abzugeben. UV-B-Filter können öllöslich oder wasserlöslich sein. Als typische UV-A-Filler kommen insbesondere Derivate des Benzoylmethans in Frage. Die UV-A und UV-B-Filter können selbstverständlich auch in Mischungen eingesetzt werden, z.B. Kombinationen aus den Derivaten des Benzoylmethans, z. B. 4-tert.-Butyl-4'-methoxydibenzoylmethan (Parsol® 1789) und 2-Cyano-3,3-phenylzimtsäure-2-ethyl-hexylester (Octocrylene) sowie Estern der Zimtsäure, vorzugsweise 4-Methoxyzimtsäure-2-ethylhexylester und/oder 4-Methoxyzimtsäurepropylester und/oder 4-Methoxyzimtsäureisoamylester. Häufig werden derartige Kombinationen mit wasserlöslichen Filtern wie z.B. 2-Phenylbenzimidazol-5-sulfonsäure und deren Alkali, Erdalkali-, Ammonium-, Alkylammonium-, Alkanolammonium- und Glucammoniumsalze kombiniert.

Neben den genannten löslichen Stoffen kommen auch unlösliche Lichtschutzpigmente, nämlich feindisperse Metalloxide in Frage. Beispiele für geeignete Metalloxide sind insbesondere Zinkoxid und Titandioxid. Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt.

Unter **biogenen Wirkstoffen** sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z. B. Prunusextrakt, Bambaranussextrakt und Vitaminkomplexe zu verstehen.

**Desodorierende Wirkstoffe** wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend eignen sich als deosodorierende Wirkstoffe u.a. keimhemmende Mittel, Enzyminhibitoren, Geruchsabsorber oder Geruchsüberdecker.

Als **Insekten-Repellentien** kommen beispielsweise N,N-Diethyl-m-toluamid, 1,2-Pentandiol oder 3-(N-n-Butyl-N-acetyl-amino)-propionsäureethylester), welches unter der Bezeichnung Insect Repellent® 3535 von der Merck KGaA vertrieben wird, sowie Butylacetylaminopropionate in Frage.

Als **Selbstbräuner** eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

Als **Konservierungsmittel** eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

Als **Parfümöle** seien genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen, Harzen und Balsamen. Weiterhin kommen tierische Rohstoffe, wie beispielsweise Zibet und Castoreum sowie synthetische Riechstoffverbindungen vom Typ der Ester, Ether, Aldehyde, Ketone, Alkohole und Kohlenwasserstoffe in Frage.

Als **Perlglanzwachse**, insbesondere für den Einsatz in tensidischen Formulierungen, kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Stabilisatoren** können Metallsalze von Fettsäuren, wie z. B. Magnesium-, Aluminium- und/oder Zinkstearat bzw. -ricinoleat eingesetzt werden.

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein.

Die erfindungsgemäßen Zubereitungen, die erfindungsgemäßen Zusammensetzungen sowie die Ester gemäß der Erfindung eignen sich insbesondere in kosmetischen und/oder pharmazeutischen Zubereitungen zur Benetzung oder Imprägnierung oder Beschichtung von Gebrauchs- und Hygienetüchem, die zur Körperreinigung und/oder zur Körperpflege eingesetzt werden.

Als Gebrauchs- und Hygienetücher seien exemplarisch genannt: Tissues, Papiere, Wipes, Vlies-Produkte, Schwämme, Puffs, Pflaster und Bandagen, die ihren Einsatz im Bereich der Hygiene und Pflege finden. Dies können sein Feuchttücher zur Baby-Hygiene und Baby-Pflege, Reinigungstücher, Gesichtreinigungstücher, Hautpflegetücher, Pflegetücher mit Wirkstoffen gegen die Hautalterung, Wipes mit Sonnenschutzformulierungen und Insektenrepellentien sowie Wipes zur dekorativen Kosmetik oder zum After-Sun-Treatment, Toiletten-Feuchttücher, Antitranspirant-Wipes, Windeln, Taschentücher, Wet Wipes, Hygieneprodukte sowie Selbstbräunungs-Wipes.

### Beispiele

### Beispiel 1: Herstellung 2-Butyloctylbulansäureester mittels Veresterung

Es wurden 344 g Buttersäure (3,9 Mol) und 852 g Isofol 12 (Butyloctanol) (4,7 Mol) zusammen mit 0,6 g Fascat® 2001 (Sn-Oxalat: 0,1 % Gew.-% bezogen auf die Gesamtzusammensetzung) 5 h auf 240 °C am Wasserabscheider erhitzt. Nach Beendigung der Wasserabscheidung wurde zunächst der Überschuss an Butyloctanol abdestilliert und anschließend das Produkt im Produkt im Vakuum destilliert (139°C bei 3 mbar). Das Produkt fiel als farbloses, dünnflüssiges Öl an.

### Rezepturen

**Tabelle 1: Öl-in Wasser-Emulsionen**

| **Inhaftsstoffe Handelsname (INCI)** | **1** | **2** | **3** | **4** | **5** |
|---|---|---|---|---|---|
| Emulgade® PL 68/50 (Cetearyl Glucoside, Cetearyl Alcohol) | 4,50 | 4,50 | 4,50 | | |
| Eumulgin® VL75 (Lauryl Glucoside, Polyglyceryl-2 Dipolyhydroxystearate, Glycerin) | | | | 4,50 | 4,50 |
| **2-Butyloctylbutansäureester** (nicht erfindungsgemäß) | 14,00 | 16,00 | | | 16,00 |
| **2-Butyloctylhexansäureester** | | | 16,00 | 12,00 | |
| Carbopol® 980 | | | | 0,30 | 0,30 |
| Lanette® 0 | | | | | |
| KOH (20%ig) | | | | 0,70 | 0,70 |
| Glycerin 99,5%ig | 3,00 | 3,00 | 3,00 | 3,00 | 3,00 |
| Formalinlsg, 37%ig | 0,15 | 0,15 | 0,15 | 0,15 | 0,15 |
| Wasser dest. | ad 100 | ad 100 | ad 100 | ad 100 | ad 100 |

**Tabelle 2: Öl-in Wasser-Emulsionen**

| **Inhaltsstoffe: Handelsname (INCi)** | **6** | **7** | **8** | **9** |
|---|---|---|---|---|
| Eumulgin® VL75 (Lauryl Glucoside, Polyglyceryl-2 Dipolyhydroxystearate, Glycerin) | 4,50 | | | |
| Eumulgin ®B2 (Ceteareth-20) | | 2,00 | 2,00 | 2,00 |
| **2-Butyloctylbutansäureester** (nicht erfindungsgemäß) | | 14,00 | 16,00 | |
| **2-Butyloctylhexansäureester** | 16,00 | | | 16,00 |
| Carbopol® 980 | 0,30 | | | |
| Lanette® 0 | | 5,00 | 5,00 | 5,00 |
| KOH (20%ig) | 0,70 | | | |
| Glycerin 99,5%ig | 3,00 | 3,00 | 3,00 | 3,00 |
| Formalinlsg. 37%ig | 0,15 | 0,15 | 0,15 | 0,15 |
| Wasser dest. | ad 100 | ad 100 | ad 100 | ad 100 |
| pH-Wert | 6,70 | 7,10 | 5,70 | 6,80 |

**Tabelle 3: Wasser-in Öl-Emulsionen**

| **Inhaltsstoffe: Handelsname (INCI)** | **10** | **11** | **12** | **13** | **14** | **15** |
|---|---|---|---|---|---|---|
| Dehymuls ®LE (PEG-30-Dipolyhydroxystearate) | 5,00 | 5,00 | 5,00 | | | |
| Dehymuls® PGPH (Polyglyceryl-2-Dipolyhydroxystearate) | | | | 4,00 | 4,00 | 4,00 |
| Lameform ®TGI (Polyglyceryl-3-Diisostearate) | | | | 2,00 | 2,00 | 2,00 |
| **2-Butyloctylbutansäureester** | 20,00 | | 18,00 | 20,00 | | 18,00 |
| **2-Butyloctylhexansäureester** | | 20,00 | | | 20,00 | |
| MgSO4*7H2O | 1,00 | 1,00 | 1,00 | 1,00 | 1,00 | 1,00 |
| Glycerin 99,5%ig | 5,00 | 5,00 | 5,00 | 5,00 | 5,00 | 5,00 |
| Formalinlsg. 37%ig | 0,15 | 0,15 | 0,15 | 0,15 | 0,15 | 0,15 |
| Wasser dest. | Ad 100 | ad 100 | ad 100 | ad 100 | ad 100 | ad 100 |

**Tabelle 4: Wasser-in Öl-Emulsionen**

| **Inhaltsstoffe Handelsname (INCI)** | **16** | **17** | **18** |
|---|---|---|---|
| Dehymuls ®LE (PEG-30-Dipolyhydroxystearate) | 4,00 | 4,00 | 4,00 |
| Lameform ®TGI (Polyglyceryl-3-Diisostearate) | 2,00 | 2,00 | 2,00 |
| 2-Butyloctylbutansäureester (nicht erfindungsgemäß) | 20,00 | | |
| 2-Butyloctylhexansäureester | | 20,00 | 15,00 |
| MgSO4*7H2O | 1,00 | 1,00 | 1,00 |
| Glycerin 99,5%ig | 5,00 | 5,00 | 5,00 |
| Formalinlsg. 37%ig | 0,15 | 0,15 | 0,15 |
| Wasser dest. | ad 100 | ad 100 | ad 100 |

**Weitere Formulierungs-Beispiele**

| Beispiel **19:** Hair Conditioner | | Beispiel **20:** Nanoemulsion | |
|---|---|---|---|
| Dehyquart®A CA (Cetrimonium Chloride) | 4.5% | Monomuls® 90 018 (Glyceryl Oleate) | 6,11% |
| | | **2-Butyloctylhexansäureester** | 17,88% |
| Lanette®O (Cetearyl Alcohol) | 4% | Eulanol®G (Octyldodecanol) | 5,97% |
| Cutina ®CP (Cetyl Palmitate) | 1% | Plantapon®LGC Sorb (Sodium Lauryl Glucose | 9,5% |
| **2-Butyloctylhexansäureester** | 1.5% | Carboxylate (and) Lauryl Glucoside) | |
| Eumulgin ®B2 (Ceteareth-20) | 0.3% | | |
| Konservierungsmittel | q.s. | Plantapon® ACG 35 (Disodium Cocoyl | 0,78% |
| Aqua demin. | ad 100 | Glutamate) | |
| | | Phenoxyethanol | 0,5% |
| | | Phenonip | 0,5% |
| | | Aqua demin. | ad 100 |

**Tabelle 5**

| **Handelsname (INCI)** | **21** | **22** | **23** | **24** | **25** | **26** | **27** |
|---|---|---|---|---|---|---|---|
| Emulgade® SE-PF (Glyceryl Stearate, Ceteareth-20, Ceteareth-12, Cetearyl Alcohol, Cetyl Palmitate) | 4,80 | | | | | | |
| Eumulgin® B2 (Ceteareth-20) | 3,70 | | 3,00 | | | | |
| Emulgade® PL-68/50 (Cetearyl Glucoside, Cetearyl Alcohol) | | | | 5,00 | | | |
| Eumulgin® SG (Sodium Stearoyl Glutamate) | | | | 0,50 | 0,20 | | |
| Eumulgin®VL 75(Lauryl Glucoside, Polyglyceryl-2 Dipolyhydroxystearate, Glycerin) | | 6,00 | | | | | 0,50 |
| Cutina® MD (Glyceryl Stearate) | | | | 2,00 | | | |
| Cutina® PES (Pentaerythrityl Distearate) | | | | | 1,00 | | |
| **2-Butyloctylbutansäureester** (nicht erfindungsgemäß) | 5,00 | 7,00 | 2,00 | 5,00 | 5,00 | 5,00 | 6,00 |
| Cetiol® 868 (Ethylhexyl Stearate) | | | 7,00 | 4,00 | | | |
| Cetiol® AB (C12-15 Alkyl Benzoate) | | 7,00 | | | | | |
| Cetiol® LC (Coco-Caprylate / Caprate) | | | | | 5,00 | 5,00 | |
| Myritol® 331 (Cocoglycerides) | 3,00 | | | | | | 10,00 |
| Myritol® 312 (Caprylic / Capric Triglyceride) | | | | 5,00 | | | |
| Myritol® 318 (Caprylic / Capric Triglyceride) | | | 7,00 | | | | |
| Dimethicone (Wacker AK 350) | | | | 0,50 | | | |
| Ethylhexyl Methoxycinnamate (Uvinul MC 80) | 5,00 | 7,50 | | | | | 7,50 |
| 4-Methylbenzylidene Camphor (Neo Helipan MBC) | 2,00 | | | | | | |
| Butyl Methoxydibenzoylmethane (Parsol 1789) | 1,50 | 3,50 | | | | | 2,00 |
| Copherol® F 1300 C (Tocopherol) | | | | 1,00 | | | 1,00 |
| Cosmedia® DC (Hydrogenated Dimer Dilinoleyl/Dimethylcarbonate Copolymer) | | | | | | | 2,00 |
| Cosmedia® SP (Sodium Polyacrylate) | | 0,50 | 0,20 | | 1,00 | 1,00 | 0,30 |
| Glycerin | 5,00 | | | 2,00 | 5,00 | | 5,00 |
| 1,3-Butylene Glycol | | 3,00 | | 2,00 | | | |
| Phenylbenzimidazole Sulfonic Acid (Neo Heliopan Hydro, 15% wässrige Lösung) | 13,30 | | | | | | |
| Methylene Bis-Benzotriazolyl Tetramethylbutylphenol (Tinosorb M) | | 5,00 | | | | | |
| Tapioca Stärke | | | | | | | |
| Wasser, Konservierungsmittel q.s. | | | | | | | |
| NaOH (10%) | pH 7,0 | pH 6,6 | pH 6,3 | pH 7,0 | pH 6,1 | pH 6,5 | pH 6,0 |

**Tabelle 6**

| **Handelsname (INCI)** | **28** | **29** | **30** | **31** |
|---|---|---|---|---|
| Dehymuls® PGPH (Polyglyceryl-2 Dipolyhydroxystearate) | 2,00 | 2,00 | | |
| Dehymuls® LE (PEG-30 Dipolyhydroxystearate) | | 2,00 | | |
| Cyclopentasiloxane, Caprylyl Dimethicone, Ethoxy Glucoside (Wacker Belsil SPG 128VP) | 12,00 | | | |
| Bienenwachs 8100 (Kahl) | 1,00 | | | |
| Zinkstearat (Zinkum N 29) | 1,00 | | | |
| Texapon® NSO (Sodium Laureth Sulfate) | | | | 34,00 |
| Dehykon® PK 45 (Cocamidopropyl Betaine) | | | | 8,00 |
| Ernulgade® NLB (Steareth-2, Ceteareth-12, Stearyl Alcohol, Ceteareth-20, Distearyl Ether) | | | | 3,00 |
| Polyquaternium-10 (Polymer JR 400) | | | | 0,20 |
| Acrylates Copolymer (Carbopol Aqua SF-1) | | | | 8,00 |
| **2-Butyloctylbutansäureester** (nicht erfindungsgemäß) | 8,00 | 6,00 | 10,00 | 3,00 |
| Cetiol® 868 (Ethylhexyl Stearate) | 7,00 | | | |
| Cetiol® A (Hexyl Laurate) | | 6,00 | | |
| Cetiol® SN (Cetearyl Isononanoate) | | 7,00 | | |
| Eutanol® G 16 (Hexyldecanol) | | 3,00 | | |
| Myritol® 331 (Coroglyrerides) | | | 31,00 | |
| Helianthus Annuus (Sonnenblumenöl) | | | 57,00 | |
| Copherol® 1250 C (Tocopheryl Acetate) | | | 1,00 | |
| Copherol® F 1300 C (Tocopherol) | 1,00 | | | |
| Glycerin | | 5,00 | | |
| 1,3-Butylene Glycol | 3,00 | | | |
| Sodium Chloride | 0,40 | | | |
| Magnesium Sulfate Heptahydrate | | 1,00 | | |
| Alcohol (Ethanol) | | 4,00 | | |
| Hydaqen® B (Bisabolol) | | | 0,50 | |
| Wasser, Konservierungsmittel q.s. | ad 100 | ad 100 | | ad 100 |

**Tabelle 7**

| **Handelsname (INCI)** | **32** | **33** | **34** |
|---|---|---|---|
| Emulgade® NLB (Steareth-2, Ceteareth-12, Stearyl Alcohol, Ceteareth-20, Distearyl Ether) | 5,00 | 5,00 | |
| Lanette® 18 (Stearyl Alcohol) | | | 14,70 |
| Cutina® HR (Hydrogenated Castor Oil) | | | 3,70 |
| **2-Butyloctylbutansäureester** (nicht erfindungsgemäß) | 6,00 | 4,50 | 23,70 |
| Cyclomethicone (Dow Corning 245) | 1,50 | 1,50 | 35,00 |
| Aluminium Chlorohydrate (Chlorhydrol 50%ig) | 40,00 | 20,00 | |
| Aluminium Zirconium Tetrachlorohydrex GLY (Rezal 36 GP) | | | 22,90 |
| Wasser | ad 100 | ad 100 | |
| Fußnoten zu Tabellen: RT = Raumtemperatur 20°C; Upm= Umdrehungen pro Minute | | | |

## Patentansprüche

1. Verwendung von Estern des 2-Butyl-1-octanols mit acyclischen C6 bis C10-Carbonsäuren in kosmetischen und/oder pharmazeutischen Zubereitungen.

2. Verwendung gemäß Anspruch 1 **dadurch gekennzeichnet, dass** als Carbonsäuren gesättigte Carbonsäuren eingesetzt werden.

3. Kosmetische und/oder pharmazeutische Zusammensetzungen enthaltend
a) wenigstens einen Ester des 2-Butyl-1-octanols mit linearen C6-C10-Carbonsäuren,
b) wenigstens einen Emulgator (b-1) und/oder Tensid (b-2) und/oder Wachskomponente (b-3) und/oder Polymer (b-4) und/oder einen weiteren Ölkörper (b-5).

## Claims

1. Use of esters of 2-butyl-1-octanol with acyclic C6-C10-carboxylic acids in cosmetic and/or pharmaceutical preparations.

2. Use according to Claim 1, **characterized in that** the carboxylic acids used are saturated carboxylic acids.

3. Cosmetic and/or pharmaceutical compositions comprising
a) at least one ester of 2-butyl-1-octanol with linear C6-C10-carboxylic acids,
b) at least one emulsifier (b-1) and/or surfactant (b-2) and/or wax component (b-3) and/or polymer (b-4) and/or a further oil body (b-5).

## Revendications

1. Utilisation d'esters du 2-butyl-1-octanol avec des acides C6-C10-carboxyliques acycliques dans des compositions cosmétiques et/ou pharmaceutiques.

2. Utilisation selon la revendication 1, **caractérisée en ce qu'**on utilise, comme acides carboxyliques, des acides carboxyliques saturés.

3. Compositions cosmétiques et/ou pharmaceutiques, contenant
a) au moins un ester du 2-butyl-1-octanol avec des acides C6-C10-carboxyliques linéaires,
b) au moins un émulsifiant (b-1) et/ou agent tensioactif (b-2) et/ou composant cireux (b-3) et/ou polymère (b-4) et/ou autre corps huileux (b-5).
